# EUROPÄISCHE PATENTANMELDUNG

(11) **EP 3 220 354 A1**
(43) Veröffentlichungstag der Anmeldung: **20.09.2017**
(21) Anmeldenummer: 17161119.7
(22) Anmeldetag: 15.03.2017
(51) Int. Cl.: G06T 7/73

(54) **VERFAHREN ZUR AUTOMATISCHEN ERKENNUNG VON ANATOMISCHEN LANDMARKEN UND VORRICHTUNG**

(30) Priorität: 15.03.2016 DE 102016204225
(71) Anmelder: Siemens Healthcare GmbH, 91052 Erlangen (DE)
(72) Erfinder: Hoffmann, Matthias, 90451 Nürnberg (DE); Strobel, Norbert, 91301 Forchheim (DE)

(57) **Zusammenfassung**

Für eine besonders schnelle und aufwandsarme Unterstützung eines Nutzers ist ein Verfahren zur automatischen Erkennung mindestens einer anatomischen Landmarke in einem Hohlorgan eines Patienten mit den folgenden Schritten vorgesehen: Bereitstellung eines medizinischen Bilddatensatzes des Hohlorgans, Ermittlung oder Bereitstellung eines dreidimensionalen Gitternetzes einer Oberfläche des Hohlorgans aus dem Bilddatensatz, Bestimmung einer Centerline des Gitternetzes durch Skelettierung, Bestimmung mindestens eines Merkmals von einer Vielzahl von Punkten auf der Centerline, Verwendung eines insbesondere auf das mindestens eine Merkmal vortrainierten Klassifikators zur Detektierung von Kandidaten für die zumindest eine anatomische Landmarke aus der Vielzahl von Punkten, Zusammenfassung der Kandidaten mit einem Abstand zueinander unterhalb einer vorgegebenen Schwelle, Verwendung von zumindest einer aus der Anatomie des Hohlorgans bestimmten Vorschrift zur Bestätigung oder Verwerfung der Kandidaten für die zumindest eine anatomische Landmarke, und Bestimmung eines oder mehrerer Kandidaten als anatomische Landmarke durch den Klassifikator. Die Erfindung umfasst außerdem eine zur Durchführung des Verfahrens vorgesehene Vorrichtung.

## Beschreibung

Die Erfindung betrifft ein Verfahren zur automatischen Erkennung mindestens einer anatomischen Landmarke in einem Hohlorgan eines Patienten gemäß dem Patentanspruch 1 sowie eine Vorrichtung zur Durchführung eines derartigen Verfahrens gemäß dem Patentanspruch 16.

Im Allgemeinen ist eine Markierung von anatomischen Merkpunkten in medizinischen Bildern eine wichtige Vorgehendweise, um z.B. Therapieplanungen vorzubereiten. Ein Beispiel aus der Elektrophysiologie (EP) ist die Position der Pulmonarvenen(PV)-Ostien und des linken atrialen Fortsatzes (LAA) in einem linken Atrium. Die Markierung der Pulmonarvenenostien erleichtert die Navigation von Kathetern, da deren Position für die automatische Planung von Katheterablationen (Koch et al.: Automatic planning of atrial fibrillation ablation lines using landmark-constrained nonrigid registration, J. Med. Imag. 1(1), 2014) verwendet werden kann und da deren Form ein wichtiges Kriterium in Bezug auf die Auswahl des zu verwendenden Kryo-Ballonkatheters ist. Ein Markieren der Bereiche und eine Anzeige in einem Röntgen-Überlagerungsbildes ist wichtig zur Vermeidung von Verletzungen durch falsches Einführen des Katheters.

Andere Beispiele aus der EP sind die Detektion gemeinsamer Ostien und die Detektion der zusätzlichen Pulmonarvene (fünfte PV). Andere Anwendungen der vorgeschlagenen Methode finden sich z.B. auf dem Gebiet der strukturellen Herzkrankheit. Dort ist die Bestimmung von Durchmessern wichtig z.B. für Klappenaustausch, z.B. bei der Aortenklappe, da die Auswahl von Größe und eventuell auch Typ der Klappe entsprechend getroffen werden kann. Ebenso kann die Methode für die Detektion von Abflüssen verwendet werden. Ein weiteres Anwendungsgebiet ist das Gebiet der abdominiellen Aortenanorysmen, insbesondere die Auswahl der passenden Stents. Zusätzliche Anwendungen sind in der Detektion von Stenosen in Blutgefäßen und Luftwegen sowie in der Detektion von Schwankungen des Durchmessers (Erweiterungen und Verengungen) im Magen-Darm-Trakt zu finden.

Für eine Therapieplanung ist es üblich, wichtige anatomische Landmarken zu markieren, einerseits weil sie kritisch sind und auf keinen Fall verengt werden dürfen (Nierenarterien in AAA Therapien, Koronararterien bei der Aortenklappenpositionierung, LAA bei EP Ablation bei Vorhofflimmern) oder weil sie wichtig für die Positionierung von Geräten sind (EP Pulmonarvenenisolierung). In der EP wird im Allgemeinen das Modell des linken Atriums unter Verwendung eines automatischen Segmentierungstools aus einem 3D CT oder MRI Volumen segmentiert. Das 3D-Modell, z.B. dargestellt als Gitternetz, wird dem Betrachter dann in einer interaktiven 3D-Ansicht gezeigt. Der Nutzer markiert eine Reihe von Punkten auf der Oberfläche des Netzes um eine Markierung des PV-Osiums und des LAAs zu erzeugen. Danach wird das markierte Modell als Teil eines Röntgen-Überlagerungs-bildes visualisiert, wodurch es dann verwendet werden kann, um die Navigation innerhalb der 3D-Kammer zu vereinfachen.

Alternativ kann ein statistisches Modell des Umrisses für die Segmentierung verwendet werden, welches Modell den Körper des LA und die Pulmonarvenen jeweils einzeln modelliert enthält. Wegen der Struktur des Modells kann die Markierung der Pulmonarvenenostien implizit aus dem Übergang vom LA-Körper auf die Pulmonarvenen abgeleitet werden (z.B. aus dem Artikel von Karim et al.: Left atrium pulmonary veins: segmentation and quantification for planning atrial fibrillation ablation, Proc. SPIE, 2009, p. 72611T ff. oder aus dem Artikel von Zheng et al.: Precise segmentation of the left atrium in C-arm CT volumens with applications to atrial fibrillation, Proc. IEEE Int. Symp. Biomed. Imaging, IEEE, p. 1421 ff., 2012). Alternativ kann auch ein halbautomatischer Ansatz verwendet werden (Rettmann et al.: Identification of the left pulmonary vein ostia using centerline tracking, Proc. SPIE., Vol. 7262, p. 726228ff., 2009). Dieses Verfahren erfordert, dass der Nutzer manuell auf jede der vier Pulmonarvenen klickt, um eine sogenannte Centerline (dreidimensionale Zentrallinie) zu berechnen. Für jeden Punkt der Centerline wird der Bereich der Schnittfläche der Pulmonarvene berechnet und der erste Punkt, ab welchem der Bereich der Schnittfläche sich signifikant vergrößert, wird als das Ostium betrachtet.

Es ist Aufgabe der vorliegenden Erfindung, ein Verfahren zur Erkennung einer anatomischen Landmarke in einem Hohlorgan eines Patienten bereitzustellen, welches ohne eine Eingabe eines Nutzers auskommt; des Weiteren ist es Aufgabe der Erfindung, eine für die Durchführung des Verfahrens geeignete Vorrichtung bereitzustellen.

Die Aufgabe wird erfindungsgemäß gelöst durch ein Verfahren zur automatischen Erkennung einer anatomischen Landmarke in einem Hohlorgan eines Patienten gemäß dem Patentanspruch 1 und von einer Vorrichtung gemäß dem Patentanspruch 16. Vorteilhafte Ausgestaltungen der Erfindung sind jeweils Gegenstand der zugehörigen Unteransprüche.

Das erfindungsgemäße Verfahren zur automatischen Erkennung mindestens einer anatomischen Landmarke in einem Hohlorgan eines Patienten weist die folgenden Schritte auf: Bereitstellung eines medizinischen Bilddatensatzes des Hohlorgans, Ermittlung oder Bereitstellung eines dreidimensionalen Gitternetzes der Oberfläche des Hohlorgans aus dem Bilddatensatz, Bestimmung einer Centerline des Gitternetzes durch Skelettierung, Bestimmung mindestens eines Merkmals für jeden von einer Vielzahl von Punkten auf der Centerline, Verwendung eines insbesondere auf das mindestens eine Merkmal vortrainierten Klassifikators zur Detektierung von Kandidaten für die zumindest eine anatomische Landmarke aus der Vielzahl von Punkten, Zusammenfassung der Kandidaten mit einem Abstand zueinander unterhalb einer vorgegebenen Schwelle, Verwendung von zumindest einer aus der Anatomie des Hohlorgans bestimmten Vorschrift zur Bestätigung oder Verwerfung der Kandidaten für die zumindest eine anatomische Landmarke, und Festlegung eines oder mehrerer Kandidaten als anatomische Landmarke. Das erfindungsgemäße Verfahren hat unter anderen die Vorteile, dass ein Eingriff eines Nutzers nicht notwendig ist, sondern alle Schritte automatisch durchgeführt werden, so dass eine Nutzerunabhängige, gleichbleibende, sehr gute Qualität und verbesserte Genauigkeit in der Erkennung von anatomischen Landmarken erzielt werden kann. Das Verfahren ist dadurch auch sehr schnell, so dass eine Vielzahl von Detektierungen in sehr kurzer Zeit erfolgen können und der klinische Ablauf beschleunigt werden kann, was wiederum den Patienten zugutekommt. Das Verfahren ist außerdem unabhängig von dem Aufnahmeverfahren der 3D-Bilder, da es nicht mit Volumen arbeitet, sondern mit 3D-Gitternetzen. Insbesondere ist das Verfahren geeignet, bei einem linken Atrium mindestens ein Pulmonarvenenostium, insbesondere alle vier bzw. fünf bzw. sechs Pulmonarvenenostien zu erkennen.

Unter einer Centerline ist eine dreidimensionale Zentrallinie zu verstehen, welche im Allgemeinen einen elliptischen Durchmesser aufweist. Z.B. aus der Veröffentlichung Telea et al.: Computing curve skeletons from medial surfaces of 3D shapes, Theory and Practice of Computer Graphics, The Eurographics Association, 2012, ist eine Methode bekannt, derartige Centerlines zu berechnen, sie werden hierin als Kurvenskelette bezeichnet.

Nach einer Ausgestaltung der Erfindung wird das Merkmal von einem Merkmal aus der Gruppe: Minimum, Maximum oder Median der Querschnittsfläche der Centerline an dem Punkt und/oder seiner Umgebung, räumlich gefiltertes Minimum, räumlich gefiltertes Maximum oder räumlich gefilterter Median der Querschnittsfläche der Centerline an dem Punkt und/oder seiner Umgebung, Änderung des Durchmessers der Centerline, maximaler Durchmesser in distaler Richtung, minimaler Durchmesser in proximaler Richtung, Position des Punktes in Bezug auf das Gravitationszentrum des Hohlorgans, Richtung der Centerline an dem Punkt, Entfernung zu dem Gravitationszentrum des Hohlorgans entlang der Centerline, Krümmung der Oberfläche des Gitternetzes entlang der Querschnittsfläche der Centerline, gebildet. Es können auch mehrere Merkmale oder auch alle genannten Merkmale der Centerline bestimmt werden. Die genannten Merkmale der Centerline können auf einfache Weise Rückschlüsse auf das Gitternetz und damit auf das Hohlorgan geben. Die Bestimmung der Merkmale ist unter anderem in der nachveröffentlichten Schrift Hoffmann et al.: Automatic detection of ostia in the left atrium, Bildverarbeitung für die Medizin (BVM), Informatik Aktuell, Springer Verlag, 2016, beschrieben. Als Punkte, für die die Merkmale bestimmt werden, können z.B. automatisch alle oder bestimmte Punkte in der Nähe von Abzweigungen der Centerline ausgewählt werden. Es können aber auch für alle Punkte auf der Centerline das oder die Merkmale berechnet werden, also die Centerline vollständig abgescannt werden.

Nach einer weiteren Ausgestaltung der Erfindung wird zusätzlich ein von der anatomischen Landmarke abhängiger Vorschlag für eine Behandlungsplanung mittels eines Klassifikators ausgewählt und ausgegeben. Bei einem solchen Vorschlag für eine Behandlungsplanung kann es sich z.B. um einen Vorschlag für eine im Bereich der anatomischen Landmarke einzuführende Vorrichtung handeln. Speziell kann z.B. im Falle eines linken Atriums und eines Pulmonarvenenostiums ein Vorschlag für einen speziell auf das entsprechende Pulmonarvenenostium passenden Katheter und/oder einen Kryoballon oder Stent ausgegeben werden. Dies erleichtert insbesondere einem Arzt das weitere Vorgehen, da er nun bereits einen Vorschlag geliefert bekommt, den er dann entsprechend seiner Erfahrung bewerten kann. Die Informationen zu den Kathetern und Kryoballons (also z.B. Größe, Marke...) können aus einer Datenbank abgefragt werden.

In vorteilhafter Weise wird zur Erkennung für jede zu erkennende anatomische Landmarke ein weiterer Klassifikator verwendet. Es wird also bei mehreren zu erkennenden Landmarken für jede Landmarke jeweils ein speziell auf die entsprechende Landmarke vortrainierter Klassifikator verwendet. Dadurch kann eine besonders gute Genauigkeit bei der Erkennung der Landmarken erzielt werden, da jeder Klassifikator spezialisiert ist.

In vorteilhafter Weise wird zur Erkennung für mehrere anatomische Landmarken ein Klassifikator verwendet. Hierdurch wird das Verfahren deutlich vereinfacht und kann besonders schnell durchgeführt werden. Es kann auch für anatomische Landmarke und zugehörigen Vorschlag für einen Behandlungsplan derselbe Klassifikator verwendet werden.

Nach einer weiteren Ausgestaltung der Erfindung wird der Klassifikator von einem Entscheidungsbaum (decision tree) gebildet. Derartige Klassifikatoren sind bekannt, leicht verfügbar, schnell und können besonders gut auf die entsprechenden Landmarken trainiert werden. Als Klassifikator kann insbesondere auch eine Stützvektormaschine (SVM = Support vector machine) oder auch ein künstliches neuronales Netzwerk verwendet werden. Der Klassifikator oder die Klassifikatoren ist/ sind insbesondere maschinen-lernend ausgebildet. Zum Training des bzw. der Klassifikatoren können z.B. sogenannte deep-learning Methoden verwendet werden. Derartige Methoden sind z.B. aus dem Artikel von Krizhevsky et al.: ImageNet classification with deep convolutional neural networks, Advances in neural information processing systems, p. 1097 ff., 2012, bekannt.

In vorteilhafter Weise für eine besonders einfache Durchführung des Verfahrens hat das dreidimensionale Gitternetz die Form eines Dreiecksnetzes. Hierbei handelt es sich um eine verbreitete und bekannte Form des Gitternetzes, welches in der Computergrafik verwendet wird, und bei dreidimensionalen medizinischen Bilddatensätzen von Hohlorganen häufig bereits vorliegt bzw. einfach erstellt werden kann. Es können auch andere Formen, z.B. Vierecksnetze, verwendet werden.

Nach einer weiteren Ausgestaltung der Erfindung wird im Falle eines linken Atriums mindestens eine Vorschrift aus der Gruppe: beide Seiten des linken Atriums besitzen ein gemeinsames Ostium, jede Seite des Atriums besitzt zwei Pulmonarvenenostien, die rechte Seite des linken Atriums besitzt drei Pulmonarvenenostien und jede Pulmonarvene besitzt nur ein Ostium verwendet, um Kandidaten für anatomische Landmarken aus der Vorauswahl zu bestätigen oder abzulehnen. Die entsprechenden Vorschriften werden entsprechend auf die Kandidaten bzw. auf die bereits gruppierten Kandidaten angewandt.

Nach einer weiteren Ausgestaltung der Erfindung wird die Centerline derart ermittelt wird, dass zuerst ein Oberflächenskelett des Hohlorgans berechnet wird und anschließend aus dem Oberflächenskelett ein Kurvenskelett gebildet. Dies ist eine bekannte, einfache Methode zur Bestimmung von Centerlines, z.B. in der Schrift Telea et al. (siehe oben) beschrieben.

Der medizinische Bilddatensatz kann z.B. von Computertomographie-Bilddaten oder von Magnetresonanztomographie-Bilddaten gebildet werden.

In vorteilhafter Weise für eine Unterstützung des Nutzers bzw. des einen therapeutischen Eingriff planenden Arztes erfolgt im Anschluss an die Bestimmung eine Anzeige der anatomischen Landmarken an einer Anzeigeeinheit. Zweckmäßigerweise werden außerdem die Gitternetzpositionen, die mit den anatomischen Landmarken der Centerline verknüpft sind, berechnet.

Die Erfindung beschreibt außerdem eine Vorrichtung zur Durchführung des Verfahrens, aufweisend eine Bearbeitungseinheit zur Bereitstellung eines medizinischen Bilddatensatzes des Hohlorgans, zur Ermittlung oder Bereitstellung eines dreidimensionalen Gitternetzes der Oberfläche des Hohlorgans aus dem Bilddatensatz, zur Bestimmung einer Centerline des Gitternetzes durch Skelettierung, zur Bestimmung mindestens eines Merkmals von einer Vielzahl von Punkten auf der Centerline, zur Anwendung eines insbesondere auf das mindestens eine Merkmal vortrainierten Klassifikators zur Detektierung von Kandidaten für die zumindest eine anatomische Landmarke aus der Vielzahl von Punkten, zur Zusammenfassung der Kandidaten mit einem Abstand zueinander unterhalb einer vorgegebenen Schwelle, zur Verwendung von zumindest einer aus der Anatomie des Hohlorgans bestimmten Vorschrift zur Bestätigung oder Verwerfung der Kandidaten für die zumindest eine anatomische Landmarke, und zur Bestimmung eines oder mehrerer Kandidaten als anatomische Landmarke durch den Klassifikator, aufweisend eine Speichereinheit zur Speicherung von Daten, eine Kommunikationseinheit zur Kommunikation mit einer Datenbank, einer Eingabeeinheit zur Eingabe von Benutzerdaten und einer Anzeigeeinheit zur Anzeige von Bilddaten.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: ein Ablauf eines erfindungsgemäßen Verfahrens; und
- FIG 2: eine Skizze einer zur Durchführung des erfindungsgemäßen Verfahrens verwendbaren Vorrichtung.

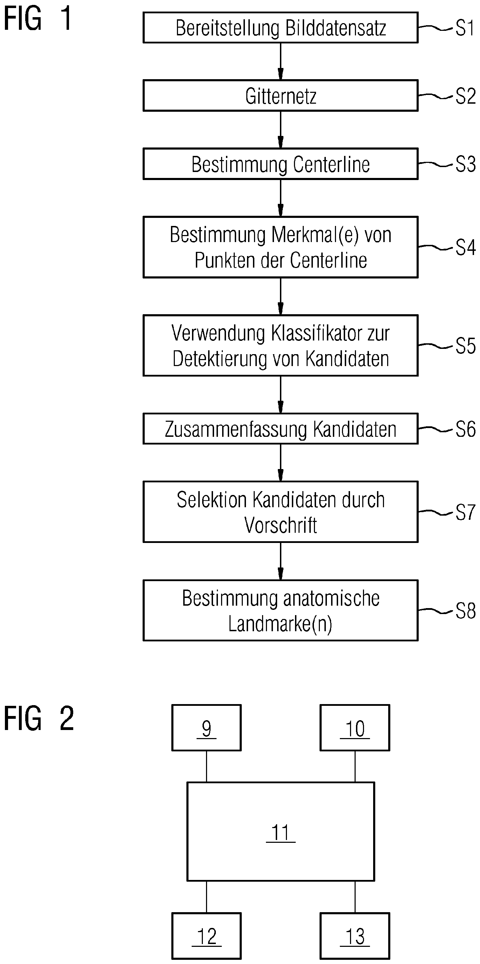

In der FIG 1 ist ein Ablauf des erfindungsgemäßen Verfahrens mit acht Schritten S1 bis S8 gezeigt. Als Beispiel für ein Hohlorgan wird das Verfahren anhand eines linken Atriums gezeigt, wobei eine Erkennung von Pulmonarvenenostien (z.B. fünf: zwei linke und drei rechte) angestrebt wird. In einem ersten Schritt S1 wird ein medizinischer 3D-Bilddatensatz eines linken Atriums eines Patienten bereitgestellt, also z.B. aus einer Datenbank oder einem Speichermedium oder direkt im Anschluss an die entsprechende Akquisition abgefragt und zur Verfügung gestellt. Bei einem solchen dreidimensionalen medizinischen Bilddatensatz kann es sich zum Beispiel um einen Computertomographie-Bilddatensatz oder einen Magnetresonanztomographie-Bilddatensatz handeln. In einem zweiten Schritt S2 wird aus dem Bildatensatz ein dreidimensionales Gitternetz, z.B. ein Dreiecksnetz (triangle mesh), der Oberfläche des Volumens berechnet. Das Gitternetz kann auch bereits zuvor erstellt worden sein und nun lediglich z.B. aus einer Datenbank oder einem Speichermedium abgefragt und zur Verfügung gestellt werden. Ein derartiges Gitternetz weist im Allgemeinen sogenannte Knoten und Kanten auf.

In einem dritten Schritt S3 wird aus dem dreidimensionalen Gitternetz eine Centerline, also eine dreidimensionale Zentrallinie, welche im Allgemeinen einen elliptischen Durchmesser aufweist, bestimmt. Eine solche Bestimmung einer Centerline wird im Allgemeinen durch Skelettierung durchgeführt. Ein geeignetes Verfahren ist z.B. in dem Artikel von Telea et al. (siehe weiter oben) beschrieben, wobei hier ein zweistufiger Ansatz gewählt wurde: zuerst wird ein Oberflächenskelett des LA (linkes Atrium) berechnet und anschließend wird aus dem Oberflächenskelett ein Kurvenskelett gebildet. Bei dem Oberflächenskelett handelt es sich um eine zweidimensionale Mannigfaltigkeit (manifold), welche aus all den Punkten aufgebaut ist, die Zentrum von durch das Netz eingetragenen Kugeln sind. Dann wird die Eigenschaft des Netzes wiederhergestellt und ein Gradientenfeld implizit auf dem Oberflächenskelett definiert. Die Punkte auf dem Oberflächenskelett werden nun iterativ entlang des Gradientenfeldes bewegt, bis sie entlang der Singularitäten des Gradientenfeldes, welche das so genannte Kurvenskelett bilden, konvergieren. Das Ergebnis ist ein sogenanntes geschrumpftes Netz (shrunken mesh).

In einem vierten Schritt S4 wird anschließend mindestens ein Merkmal für jeden einer Vielzahl von Punkten auf der Centerline bestimmt. Das Merkmal wird dabei von einem Merkmal aus der Gruppe: Minimum, Maximum oder Median der Querschnittsfläche der Centerline an dem Punkt und/oder seiner Umgebung, räumlich gefiltertes Minimum, räumlich gefiltertes Maximum oder räumlich gefilterter Median der Querschnittsfläche der Centerline an dem Punkt und/oder seiner Umgebung, Änderung des Durchmessers der Centerline, maximaler Durchmesser in distaler Richtung, minimaler Durchmesser in proximaler Richtung, Position des Punktes in Bezug auf das Gravitationszentrum des Hohlorgans, Richtung der Centerline an dem Punkt, Entfernung zu dem Gravitationszentrum des Hohlorgans entlang der Centerline, Krümmung der Oberfläche des Gitternetzes entlang der Querschnittsfläche der Centerline, gebildet. Es können auch mehrere Merkmale oder auch alle genannten Merkmale der Centerline bestimmt werden. Als Punkte, für die die Merkmale bestimmt werden, können z.B. automatisch Punkte in der Nähe von Abzweigungen der Centerline ausgewählt werden. Es können aber auch für alle Punkte auf der Centerline das oder die Merkmale berechnet werden, also die Centerline vollständig abgescannt werden.

Es kann z.B. zuerst der Radius oder Durchmesser der Pulmonarvene bestimmt werden. Dieser ist im Allgemeinen elliptisch. Es kann die Querschnittsfläche an dem Punkt und den Normalenvektor bestimmt werden, anschließend die Schnittfläche der Pulmonarvene mit dieser Querschnittsfläche und dann kann der Median des Abstandes von dem Punkt zu den sich daraus ergebenden Punkten der Pulmonarvene bestimmt werden. Danach kann eine räumliche Median-Filterung auf die Radien entlang der Pulmonarvene angewendet werden. Da das Pulmonarvenenostium dadurch charakterisiert ist, dass sich der Radius/Durchmesser in Richtung des Zentrums des linken Atriums vergrößert, werden auch die Radien bzw. Durchmesser der Umgebung der Punkte verwendet. Zur Abschätzung der Vergrößerung können auch die Ableitungen des Radius/ Durchmessers verwendet werden. Um sicherzugehen, dass eine starke Vergrößerung an einem Punkt nicht auf ein lokales Minimum im Durchmesser der Pulmonarvene zurückzuführen ist, kann auch der maximale Radius/ Durchmesser in distaler Richtung bestimmt werden. Um sicherzugehen, dass das Pulmonarvenenostium nicht mit einer lokalen Erweiterung verwechselt wird, kann auch der minimale Radius/ Durchmesser in proximaler Richtung bestimmt werden. Außerdem können auch der Normalenvektor und der Abstand zum Zentrum des linken Atriums verwendet werden.

In einem fünften Schritt S5 wird mindestens ein auf das mindestens eine Merkmal vortrainierter Klassifikator zur Detektierung von Kandidaten für die zumindest eine anatomische Landmarke aus der Vielzahl von Punkten verwendet. Bei dem Klassifikator kann es sich zum Beispiel um einen Entscheidungsbaum (decision tree) handeln. Es ist sinnvoll, den oder die Klassifikatoren anhand einer möglichst großen Anzahl an Beispielen vorzutrainieren, damit er ein möglichst akkurates Ergebnis liefert. Das Training kann z.B. durch sogenannte deep learning Methoden durchgeführt werden. Es kann vorgesehen sein, zur Erkennung von mehreren Pulmonarvenenostien für das jeweilige Ostium jeder einzelnen PV (z.B. linke obere, linke untere, rechte obere, rechte untere, gemeinsame und Zusatz-PV) einen individuellen Klassifikator zu verwenden, es kann aber für mehrere Pulmonarvenenostien auch nur ein einziger Klassifikator verwendet werden.

Es kann außerdem in diesem Zusammenhang für jeden Kandidaten für die Landmarke z.B. auf der Basis der anatomischen Gegebenheiten ein entsprechender Vorschlag für einen Therapieplan von demselben Klassifikator oder einem individuellen Klassifikator ermittelt werden. Ein Beispiel bei der Ermittlung von Kandidaten für Pulmonarvenenostien kann in diesem Zusammenhang etwa die Art/Größe und/oder der Typ eines zu verwendenden Katheters oder eines Kryoballons vorgeschlagen werden. Auch Vorschläge zu Stents und anderen medizinischen Gerätschaften können umfasst sein. Dieser optionale Schritt ist in der FIG 1 nicht gezeigt.

In einem sechsten Schritt S6 können von dem oder den Klassifikatoren ermittelte Kandidaten zusammengefasst werden (geclustert). Dies kann z.B. derart durchgeführt werden, dass Kandidaten mit einem Abstand zueinander unterhalb einer vorher festgelegten Schwelle zusammengefasst werden.

In einem siebten Schritt S7 wird zumindest eine aus der Anatomie des LAs bestimmte Vorschrift zur Bestätigung oder Verwerfung von Kandidaten für die Pulmonarvenenostien verwendet. Solche Vorschriften können z.B. lauten, dass pro Pulmonarvene nur ein einziges Ostium möglich ist oder dass eine bestimmte Seite des LA zwei Ostien oder drei Ostien (rechte Seite) hat oder beide Seiten ein gemeinsames Ostium haben.

In Bezug auf die übrigen Kandidaten bzw. Cluster von Kandidaten wird dann in einem achten Schritt S8 z.B. von dem oder den Klassifikatoren ein oder mehrerer Kandidaten oder Cluster als Pulmonarvenenostium/ostien festgelegt. Es kann z.B. das größte Cluster von Kandidaten auf der rechten Seite des LA als erstes rechtes Pulmonarvenenostium, das größte Cluster von Kandidaten auf der linken Seite als erstes linkes Pulmonarvenenostium festgelegt werden. Das jeweilige zweitgrößte Cluster ist dann das zweite linke und das zweite rechte Pulmonarvenenostium. Gibt es auf der rechten Seite des LA noch ein Cluster, so handelt es sich hierbei um das Ostium des Zusatzes (LAA).

Anschließend (nicht in der FIG 1 gezeigt) wird das Ergebnis des Verfahrens, also z.B. die ermittelten Pulmonarvenenostien einem Benutzer oder Arzt an einer Anzeigeeinheit angezeigt. Zusätzlich (auch nicht in der FIG 1 gezeigt) können auch die entsprechenden Vorschläge für Therapiepläne oder Vorschläge für zu verwendende Therapiewerkzeuge oder Geräte (z.B. Typ des Katheters und Kryoballons) angezeigt werden.

Des Weiteren kann anschließend eine Visualisierung der anatomischen Landmarken mit Live-Bildern von medizinischen Bildgebungseinrichtungen durchgeführt werden. So können z.B. Live-Fluoroskopiebilder durch das Dreiecksnetz und die anatomischen Landmarken (linkes Atrium mit Pulmonarvenenostien) überlagert angezeigt werden.

In der FIG 2 ist eine Vorrichtung zur Durchführung des erfindungsgemäßen Verfahrens gezeigt. Hierbei kann es sich z.B. um eine Verarbeitungseinheit 11 handeln, die mit einer Kommunikationseinheit 9, einer Speichereinheit 10, einer Anzeigeeinheit 12 und einer Eingabeeinheit 13 in Verbindung steht.

Die Erfindung beschreibt ein automatisches Verfahren zur Detektierung von anatomischen Merkpunkten, welche auch zur Unterstützung der Therapieplanung verwendet werden können, z.B. durch Vorschläge zur Auswahl von entsprechenden Gerätschaften und deren Positionierung. Das Atrium wird bevorzugt als Dreiecksnetz dargestellt ist, dadurch ist das Verfahren unabhängig von der Modalität, mit der der medizinische Bilddatensatz aufgenommen wurde und auch unabhängig von dem Segmentierungswerkzeug.

Die Erfindung lässt sich in folgender Weise kurz zusammenfassen: Für eine besonders schnelle und aufwandsarme Unterstützung eines Nutzers ist ein Verfahren zur automatischen Erkennung mindestens einer anatomischen Landmarke in einem Hohlorgan eines Patienten mit den folgenden Schritten vorgesehen: Bereitstellung eines medizinischen Bilddatensatzes des Hohlorgans, Ermittlung oder Bereitstellung eines dreidimensionalen Gitternetzes des Hohlorgans aus dem Bilddatensatz, Bestimmung einer Centerline des Gitternetzes durch Skelettierung, Bestimmung mindestens eines Merkmals von einer Vielzahl von Punkten auf der Centerline, Verwendung eines insbesondere auf das mindestens eine Merkmal vortrainierten Klassifikators zur Detektierung von Kandidaten für die zumindest eine anatomische Landmarke aus der Vielzahl von Punkten, Zusammenfassung der Kandidaten mit einem Abstand zueinander unterhalb einer vorgegebenen Schwelle, Verwendung von zumindest einer aus der Anatomie des Hohlorgans bestimmten Vorschrift zur Bestätigung oder Verwerfung der Kandidaten für die zumindest eine anatomische Landmarke, und Festlegung eines oder mehrerer Kandidaten als anatomische Landmarke z.B. durch den Klassifikator.

## Patentansprüche

1. Verfahren zur automatischen Erkennung mindestens einer anatomischen Landmarke in einem Hohlorgan eines Patienten mit den folgenden Schritten:
• Bereitstellung eines medizinischen Bilddatensatzes des Hohlorgans (S1),
• Ermittlung oder Bereitstellung eines dreidimensionalen Gitternetzes einer Oberfläche des Hohlorgans aus dem Bilddatensatz (S2),
• Bestimmung einer Centerline des Gitternetzes durch Skelettierung (S3),
• Bestimmung mindestens eines Merkmals von einer Vielzahl von Punkten auf der Centerline (S4),
• Verwendung eines insbesondere auf das mindestens eine Merkmal vortrainierten Klassifikators zur Detektierung von Kandidaten für die zumindest eine anatomische Landmarke aus der Vielzahl von Punkten (S5),
• Zusammenfassung der Kandidaten, insbesondere mit einem Abstand zueinander unterhalb einer vorgegebenen Schwelle (S6),
• Verwendung von zumindest einer aus der Anatomie des Hohlorgans bestimmten Vorschrift zur Bestätigung oder Verwerfung der Kandidaten für die zumindest eine anatomische Landmarke (S7), und
• Festlegung eines oder mehrerer Kandidaten als anatomische Landmarke (S8).

2. Verfahren nach Anspruch 1, wobei das Merkmal von einem Merkmal aus der Gruppe: Minimum, Maximum oder Median der Querschnittsfläche der Centerline an dem Punkt und/oder seiner Umgebung, räumlich gefiltertes Minimum, räumlich gefiltertes Maximum oder räumlich gefilterter Median der Querschnittsfläche der Centerline an dem Punkt und/oder seiner Umgebung, Änderung des Durchmessers der Centerline, maximaler Durchmesser in distaler Richtung, minimaler Durchmesser in proximaler Richtung, Position des Punktes in Bezug auf das Gravitationszentrum des Hohlorgans, Richtung der Centerline an dem Punkt, Entfernung zu dem Gravitationszentrum des Hohlorgans entlang der Centerline, Krümmung der Oberfläche des Gitternetzes entlang der Querschnittsfläche der Centerline gebildet wird.

3. Verfahren nach einem der vorangehenden Ansprüche, wobei zusätzlich ein von der anatomischen Landmarke abhängiger Vorschlag für eine Behandlungsplanung mittels eines Klassifikators ausgewählt und ausgegeben wird.

4. Verfahren nach einem der vorangehenden Ansprüche, wobei das Hohlorgan von einem linken Atrium und die mindestens eine anatomische Landmarke von einem Pulmonarvenenostium gebildet werden.

5. Verfahren nach Anspruch 4, wobei mehrere Pulmonarvenenostien erkannt werden.

6. Verfahren nach einem der vorangehenden Ansprüche, wobei zur Erkennung für jede zu erkennende anatomische Landmarke ein weiterer Klassifikator verwendet wird.

7. Verfahren nach einem der vorangehenden Ansprüche, wobei zur Erkennung für mehrere anatomische Landmarken ein Klassifikator verwendet wird.

8. Verfahren nach einem der vorangehenden Ansprüche, wobei der Klassifikator von einem Entscheidungsbaum gebildet wird.

9. Verfahren nach einem der vorangehenden Ansprüche, wobei das dreidimensionale Gitternetz die Form eines Dreiecksnetzes hat.

10. Verfahren nach Anspruch 4, wobei mindestens eine Vorschrift aus der Gruppe: beide Seiten des linken Atriums besitzen ein gemeinsames Ostium, jede Seite des Atriums besitzt zwei Pulmonarvenenostien, die rechte Seite des linken Atriums besitzt drei Pulmonarvenenostien und jede Pulmonarvene besitzt nur ein Ostium verwendet wird.

11. Verfahren nach einem der vorangehenden Ansprüche, wobei die Centerline derart ermittelt wird, dass zuerst ein Oberflächenskelett des Hohlorgans berechnet wird und anschließend aus dem Oberflächenskelett ein Kurvenskelett gebildet wird.

12. Verfahren nach einem der vorangehenden Ansprüche, wobei der medizinische Bilddatensatz von Computertomographie-Bilddaten oder von Magnetresonanztomographie-Bilddaten gebildet wird.

13. Verfahren nach einem der vorangehenden Ansprüche, wobei der Klassifikator maschinen-lernend ausgebildet ist.

14. Verfahren nach einem der vorangehenden Ansprüche, wobei im Anschluss an die Bestimmung eine Anzeige der anatomischen Landmarken an einer Anzeigeeinheit erfolgt.

15. Verfahren nach einem der vorangehenden Ansprüche, wobei die Gitternetzpositionen, die mit den anatomischen Landmarken der Centerline verknüpft sind, berechnet werden.

16. Vorrichtung zur Durchführung eines Verfahrens nach einem der Ansprüche 1 bis 15, aufweisend eine Bearbeitungseinheit zur Bereitstellung eines medizinischen Bilddatensatzes des Hohlorgans, zur Ermittlung oder Bereitstellung eines dreidimensionalen Gitternetzes einer Oberfläche des Hohlorgans aus dem Bilddatensatz, zur Bestimmung einer Centerline des Gitternetzes durch Skelettierung, zur Bestimmung mindestens eines Merkmals von einer Vielzahl von Punkten auf der Centerline, zur Anwendung eines insbesondere auf das mindestens eine Merkmal vortrainierten Klassifikators zur Detektierung von Kandidaten für die zumindest eine anatomische Landmarke aus der Vielzahl von Punkten, zur Zusammenfassung der Kandidaten mit einem Abstand zueinander unterhalb einer vorgegebenen Schwelle, zur Verwendung von zumindest einer aus der Anatomie des Hohlorgans bestimmten Vorschrift zur Bestätigung oder Verwerfung der Kandidaten für die zumindest eine anatomische Landmarke, und zur Bestimmung eines oder mehrerer Kandidaten als anatomische Landmarke durch den Klassifikator, aufweisend eine Speichereinheit zur Speicherung von Daten, eine Kommunikationseinheit zur Kommunikation mit einer Datenbank, einer Eingabeeinheit zur Eingabe von Benutzerdaten und einer Anzeigeeinheit zur Anzeige von Bilddaten.
